# EUROPÄISCHE PATENTANMELDUNG

(11) **EP 0 610 554 A2**
(43) Veröffentlichungstag der Anmeldung: **17.08.1994**
(21) Anmeldenummer: 93117553.3
(22) Anmeldetag: 29.10.1993
(51) Int. Cl.: G01N 33/00, G01N 31/00

(54) **Vorrichtung und Verfahren zur Ermittlung des NO-Gehaltes**

(30) Priorität: 02.11.1992 DE 4236944
(71) Anmelder: Termin, Andreas Dr., D-79737 Herrischried (DE)
(72) Erfinder: Termin, Andreas Dr., D-79737 Herrischried (DE)
(74) Vertreter: Termin, Erich

(57) **Zusammenfassung**

Vorliegende Erfindung betrifft eine Vorrichtung und ein Verfahren zur Ermittlung des NO-Gehaltes in Flüssigkeiten, min Hilfe eines Inertträgergases, das dadurch gekennzeichnet ist, dass die Vorrichtung aus einem Mikroreaktionsteil Fig. 1 besteht, das gegliedert ist, in den Inertgaseinleitstutzen 1, das Mikroreaktionsgefäss 2 mit Fritteboden 3, mindestens einem Eintragsstutzen für die zu analysierende Flüssigkeit 4,sowie einem kühlbaren Austragstutzen für das mit NO beladene Inertgas 5 und einem Detektor 6 mittels dem der NO-Gehalt der analysierten Flüssigkeit ermittelt und ausgewertet wird wobei die zu analysierende Flüssigkeit das NO schon überwiegend in der Form von NO₂⁻
Ionen enthält die zu NO zurückoxidiert werden müssen,mittels einem Reduktionsgemisch,bestehend aus einer Lösung von 1,1' Dimethylferocen in Acetonitril in saurem Medium, vorzugsweise der Perchlorsäure.

## Beschreibung

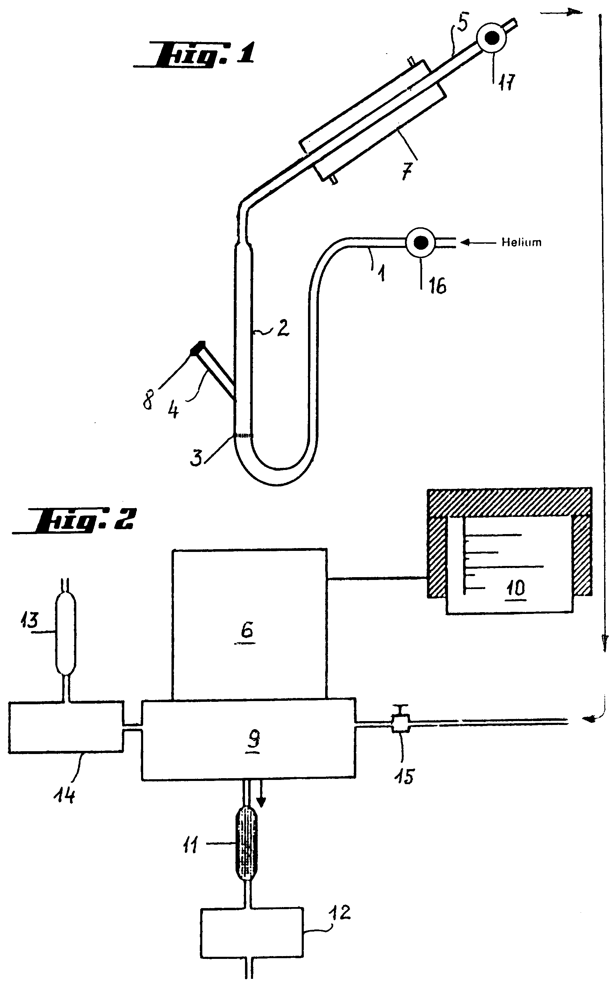

Vorliegende Erfindung betrifft eine Vorrichtung und ein Verfahren zur Ermittlung des NO-Gehaltes in Flüssigkeiten, mit Hilfe eines Inertträgergases, das dadurch gekennzeichnet ist, dass die Vorrichtung aus einem Mikroreaktionsteil Fig. 1 besteht, das gegliedert ist, in den Inertgaseinleitstutzen 1, das Mikroreaktionsgefäss 2 mit Fritteboden 3, mindestens einem Eintragsstutzen für die zu analysierende Flüssigkeit 4, sowie einem kühlbaren Austragstutzen für das mit NO beladene Inertgas 5 und einem Detektor 6 mittels dem der NO-Gehalt der analysierten Flüssigkeit ermittelt und ausgewertet wird.

Stichstoffoxid ist ein sehr reaktives Gas, das insbesondere in Anwesenheit von Sauerstoff sehr leicht zum NO₂⁻ -Ion oxidiert. Um den exakten Ursprungsgehalt von NO in Flüssigkeiten zu ermitteln,ist es erforderlich,das intermediär entstandene Nitrit wieder in NO zu reduzieren.
Der Erfindung liegt die Aufgabe zugrunde ein besonders mildes Reduktionsmittel anzuwenden bei besonders milden Bedingungen.
Insbesondere deshalb,weil physiologische Flüssigkeiten analysiert werden die zum Beispiel als Eluat aus einer Eluierung frischer, organischer Zellen gewonnen werden.

Solche physiologische Flüssigkeiten enthalten gegebenenfalls noch verschiedene organische Bestandteile die einer Reduzierung unterliegen könnten.
Hinzu kommt, dass in einem Mikroreaktionsgefäss das Reduktionsmittelangebot begrenzt ist.

Nach dem Stande der Technik wird hierzu ein Reaktionsgefäss mit siedender Essigsäure mit 1% NaJ verwendet.
Als Kontrollstandarts dienen NO/Wasserlösungen.
Mit konstantem Druck wird Helium in die Lösung eingeleitet welches das freigestzte NO in den NO-Detektor 6 leitet.

Eine basische Kühlfalle verhindert, dass saure Dämpfe in den NO-Detektor gelangen können.

Dieses System hat folgende Nachteile.
a) Das Reaktionsgefäss muß beheizt werden
b) Die Temperatur im Reaktionsgefäss muß genau eingehalten werden um Schwankungen des Grundsignals zu verhindern
c) Die heissen Essigsäuredämpfe müssen gekühlt werden und danach muß der Gasstrom durch eine alkalische Kühlfalle geleitet werden. Dadurch wird das Gesamtvolumen der Vorrichtung erheblich ausgeweitet, was die NO-Konzentration im Gasstrom erniedrigt und somit Messgenauigkeit mindert.
d) Es müssen aufwendige Standartkurven erstellt werden.

Ziel des erfinderischen Reduktionsverfahrens war es somit, alle diese Unzulänglichkeiten auszuschalten, das Verfahren so zu vereinfachen dass bei Raumtemperatur gearbeitet werden kann, um Meßschwankungen abzubauen und eine hohe Sensibilität des Gesamtverfahrens durch ein möglichst kleines Volumen der Vorrichtung zu gewährleisten.

Die Aufgabenstellung wurde gelöst, durch ein Verfahren zur Reduktion von NO₂⁻ -Ionen, in Flüssigkeiten die Stickstoffoxid enthalten, zu Stickstoffoxid, das dadurch gekennzeichnet ist, dass das Verfahren in der Vorrichtung gemäss der Fig. 1 abläuft, bei einer Temperatur zwischen 5^{o} bis 50^{o}C, mittels einer Reduktionslösung bestehend, aus 1,1' Dimethylferocen in Acetonitril, in saurem Medium, und die Durchmischung des Reaktionsgemisches mittels einem Inertgasstrom erfolgt, der das Reaktionsgemisch über eine Frite durchströmt, wobei das Inertgas gleichzeitig als Trägergas für das Stickstoffoxidgas dient, das über den Austragstutzen 5 in den Detektor 6 transportiert und als Gesamtstickstoffoxid ermittelt wird.

Bevorzugtes saures Medium ist ca 70%-ige Perchlorsäure.
Der bevorzugte Arbeitstemperaturbereich ist der der Raumtemperatur.
Durch die niedrige Reaktionstemperatur und die Verwendeten relativ hochsiedenden flüssigen Reaktionsmedien sowie den sehr geringen Volumendurchfluss des Trägerinertgases durch das Reaktionsgemisch,wird der Dampfpartialdruck des Reaktionsgemisches nur so gering erhöht, dass dieser unberücksichtigt bleiben kann.

Diese Umstände wirken sich auf das Gesamtkonzept, Vorrichtung und Verfahren, vorteilhaft aus, da unerwünschte Solteilchen nicht in den Detektionsbereich der Apparatur Fig. 2, gelangen.

Volumenbeanspruchende Kühlzonen erübrigen sich.

Der Austragstutzen 5 ist deshalb nur ein Durchlaufrohr, umgeben mit einem Kühlmantel 7.
Gekühlt wird durch üblichen Kühlwasserdurchlauf durch den Kühlmantel.
Inertgase die zur Anwendung kommen sind die üblichen Edelgase, bevorzugt Helium.

Ein weiterer Vorteil des neuen Verfahrens zur Untersuchung von Flüssigkeiten, insbesondere physiologischer Flüssigkeiten,erhalten als Eluat organischer Zellen, ist es, dass die auf den Stickstoffoxidgehalt zu analysierende Flüssigkeit über einen der Eintragstutzen 4 in die Vorrichtung der Fig. 1 durch den Stopfen 8, direkt in das Reaktionsmedium in Gefäss 2 injiziert wird.
Dies verhindert Fehler durch eine sonst mögliche Oberflächenhaftung der Flüssigkeit an den Glasteilen.

Darüberhinaus wird gemäss dem neuen Verfahren eine stabile Natriumnitritlösung als Vergleichsstandart eingesetzt.

Das im neuen Verfahren eingesetzte 1,1' Dimethylferocen/Acetonitril/Perchlorsäure Reduktionsmedium, mit vorzugsweise 1%-iger Perchlorsäurekonzentration im Gemisch, hat auch den Vorteil,dass es durch Verdünnungen kaum beeinflusst wird.

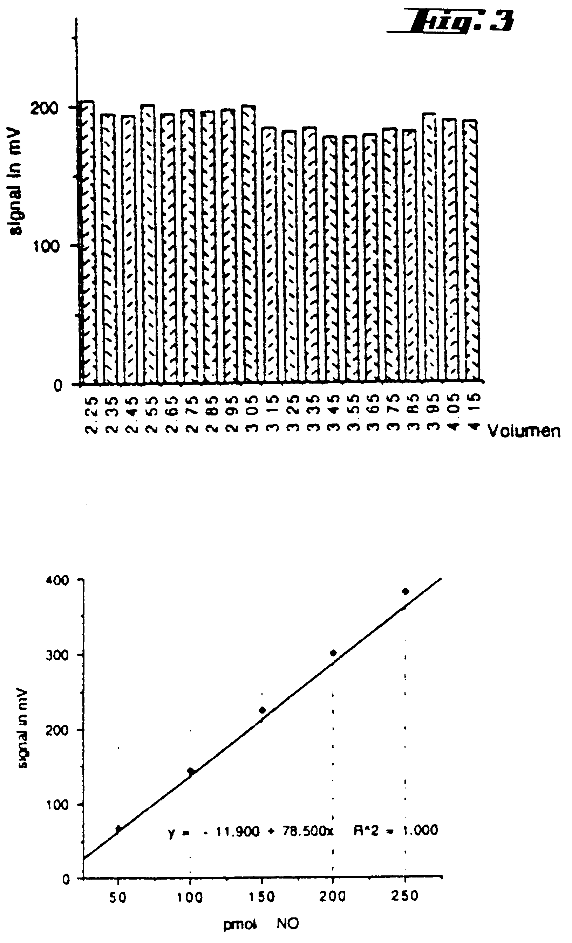

Figur 3 zeigt eine Messreihe, bei der zu 2,15 ml Reduktionsmedium jeweils 500 pmol Natriumnitrit gelöst in 100 ul Wasser zugegeben wurden. Das Signal ausgedrückt in mV bleibt für die jeweilige messung praktisch gleich.

Die Detektierung und quantitative Auswertung der Stickstoffoxidwerte die zu ermitteln sind kann nach verschiedenen Methoden erfolgen.

Der Stand der Technik kennt Methoden zur Detektierung von Stickstoffoxid, so zum Beispiel als Farbkomplex mit Hilfe von Sulfanilsäure (Diazotierung durch NO und Kopplung mit N(1-Naphthyldiamin) oder mit Oxyhemoglobin, ESR-spectroskopisch, massenspektroskopisch, mittels Spin-Traps (R), elektrochemisch (R) und mittels Chemilumineszenz.

Die Chemilumineszenzmethode bedient sich der Reaktion von NO mit Ozon zu NO₂* und O₂. Beim anschliessenden Zerfall wird Licht im Bereich von 600 bis 875 nm freigesetzt, das mittels einer Photozelle gemessen wird.

Folgende Beschreibung und Beispiele illustrieren die Erfindung im Detail.
- Fig.1: zeigt eine Vorrichtung zur Reduktion von NO/NO₂⁻-haltigen Flüssigkeiten und zur Freisetzung des Gesamt-NO-Gehalts und Ableitung des Gesamtstickstoffoxids in den Detektor und Recorderteil Fig.2 zur quantitativen Ermittlung desselben.
- Fig.2: zeigt den NO-Detektor mit Auswerter und Recorder
- Fig.3: zeigt die Unbeeinflußbarkeit der Reduktionslösung durch Verdünnungen.

### Indicesbedeutungen

- 1: Inertgaseinleitstutzen
- 2: Mikroreaktionsgefäss
- 3: Friteboden
- 4: Eintragstutzen für die zu analysierende Flüssigkeit
- 5: Austragstutzen für das mit NO beladene Inertgas
- 6: NO-Detektor
- 7: Kühlmantel
- 8: Injektionsseptum
- 9: Chemilumineszenzgenerator
- 10: Recorder zur NO Auswertung
- 11: Aktivkohlefilter
- 12: Vacuumpumpe
- 13: Sauerstoff
- 14: Ozongenerator
- 15: Regulierventil
- 16: Teflon- bzw. teflonisiertes Einlassventil
- 17: Teflon- bzw. teflonisiertes Auslassventil

### Beispiel 1

Die NO-Freisetzung von Gefässendothelzellen wird ermittelt. Hierzu werden frisch gewonnene Zellen verwendet.
Die Endothelzellen werden mit Kollagenase aus der Innenwand von Rinderaorten abgelöst und danach auf Mikroträger-Unterlagen transferiert.
Nach Bestimmung der Zelldichte werden definierte Zellaliquots auf einen Filter mit 105 um Porengröße aufgebracht und mit 37^{o}C warmer Krebs-Henseleit-Lösung superperfundiert. Das Filtrat (Eluat) wird zur Bestimmung der NO-Konzentration in der Vorrichtung Fig.1 und Detektion in Fig.2 eingesetzt.

Da die NO-Bestimmung bei Normalbedingungen erfolgt. Es werden keine Präventivmassnahmen bezüglich der Oxidation des NO zu NO₂⁻ getroffen. Deshalb muß,um das GesamtStickstoffoxid bestimmen zu können, das zwischenzeitlich entstandene NO₂⁻ zu NO zurückreduziert werden.

Dazu dient eine Reduktionslösung bestehend aus 30 mg 1,1' Dimethylferocen gelöst in 3 ml Acetonitril-HPLC-rein die angesäuert wird mit 49 ul 70%-iger Perchlorsäure.

2 ml dieser Lösung werden durch den Eintragstutzen 4 in die Vorrichtung Fig.1 injiziert. Davor wurde das Einlassventil 16 geöffnet und Helium in die Vorrichtung, mit einer Durchströmungsrate von 35 ml pro Minute, eingeleitet.
Gleichzeitig wird das Auslassventil 17 und das Regulierventil 15 geöffnet.
200 ul des , wie oben beschrieben gewonnenen Filtrats (Eluats),werden nun mittels einer Einmalinjektionsspritze durch das Injektionsseptum 8 über den Eintragstutzen 4 direkt in die Reduktionslösung im Mikroreaktionsgefäss 2 injiziert. Das analysierte Volumen enthält Stickstoffoxid in einer Menge, wie es von 120 Millionen frisch gewonnener Endothelzellen produziert wird.
Das Kühlwasser im Kühlmantel 7 hat eine Temperatur von 10^{o}C.

Über den Austragstutzen 5 gelangt das zu bestimmende NO, getragen vom Heliumstrom, in den Chemilumineszenzgenerator 9 in dem entsprechend der NO-Menge ein entsprechendes Lichtlumen der Wellenlänge 600-875 nm freigesetzt wird, das im NO-Detektor 6 als NO detektiert und auf dem Recorder 10 erfasst wird.
Ozongenerator 14, Aktivkohlefilter 14 und die Vacuumpumpe 12 sind Hilfsaggregate zur Analysierung mit Hilfe der Chemilumineszenzmethode und des Durchströmungsprinzips der Analysenmethode.
Die NO-Menge wird in pmol/ml angegeben.
Fünf verschiedene Versuche ergaben folgende NO-Werte ausgedrückt in pmol auf eingesetztes Analysensubstrat (Eluat)

| Versuch | |
|---|---|
| 1 | 232 |
| 2 | 162 |
| 3 | 874 |
| 4 | 675 |
| 5 | 426 |

Einige beispielhafte Dimensionsdaten veranschaulichen die Volumeninhaltsminimierung der Vorrichtung gemäss Fig. 1 Gesamtvolumen, der Vorrichtung bis zum Einlauf in den Austragstutzen 5, 35 ml, des Mikroreaktionsgefässes 2 ab der Glasfrite bis zum Einlauf in den Austragstutzen 5, 20 ml, Ein- und Auslaufstutzendurchmesser innen 4 mm, Innendurchmesser des Mikroreaktionsgefässes 2, 20 mm.
Mittels NO-Standartkurven,erstellt auf der Basis von exakte Mengen an Natriumnitrit enthaltenden Lösungen, das nach dem beschriebenen erfinderischen Verfahren zu NO reduziert wurde,konnte die hohe Messgenauigkeit und exakte Reproduzierbarkeit des neuen Verfahren bestätigt werden, wie dies in Fig. 4 gezeigt wird.

## Patentansprüche

1. Vorrichtung zur Ermittlung des NO-Gehalts in Flüssigkeiten mit Hilfe eines Inertträgergases, dadurch gekennzeichnet, dass die Vorrichtung aus einem Mikroreaktionsteil (Fig.1) besteht, das gegliedert ist, in den Inertgaseinleitstutzen (1), ein Mikroreaktionsgefäss (2) mit Fritteboden (3), mindestens einem Eintragstutzen (4) mit Injektionsseptum (8) für den Eintrag der zu analysierenden Flüssigkeit,sowie einem kühlbaren Austragstutzen (5) für das mit NO beladene Inertgas, und einem Detektor (6) mittels dem der NO-Gehalt der analysierten Flüssigkeit ermittelt und ausgewertet wird.

2. Vorrichtung nach Anspruch 1, dadurch gekennzeichnet, dass die Gaseintragstutzen (1) und der Austragstutzen (5) mit Ventilen versehen sind, die aus Polyterafluorethylen bestehen oder mit einem solchen Material ummantelt sind (16,17) und das Mikroreaktionsgefäss (2) ein Innenvolumen von weniger oder bis 2o ml hat und die Vorrichtung aus Glas besteht.

3. Verfahren zur Reduktion von NO₂⁻ -Ionen,in Flüssigkeiten die Stickstoffoxid enthalten, zu Stickstoffoxid, das dadurch gekennzeichnet ist, dass das Verfahren in der Vorrichtung gemäss der Fig. 1 abläuft, bei einer Temperatur zwischen 5^{o} bis 50^{o}C, mittels einer Reduktionslösung bestehend, aus 1,1' Dimethylferocen in Acetonitril, in saurem Medium, und die Durchmischung des Reaktionsgemisches mittels einem Inertgasstrom erfolgt, der das Reaktionsgemisch über eine Frite durchströmt, wobei das Inertgas gleichzeitig als Trägergas für das Stickstoffoxidgas dient, das über den Austragstutzen 5 in den Detektor 6 transportiert und als Gesamtstickstoffoxid ermittelt wird.

4. Verfahren nach Anspruch 3, dadurch gekennzeichnet, dass zur Sauerstellung des Reduktionsmedium 70%-ige Perchlorsäure eingesetzt wird und die Reduktion bei Raumtemperatur durchgeführt wird.

5. Verfahren nach den Ansprüchen 3 und 4, dadurch gekennzeichnet, dass die zu reduzierenden Flüssigkeiten physiologische Flüssigkeiten sind, gewonnen als Eluat aus der Eluierung von frischen organischen Zellen.

6. Verfahren nach den Ansprüchen 3 bis 5, dadurch gekennzeichnet, dass die zu analysierende Flüssigkeit direkt in das Reduktionsmedium injiziert wird.
